(19) **Europäisches Patentamt**
**European Patent Office**
**Office européen des brevets**

(11) **EP 3 593 900 A1**

(12) **EUROPEAN PATENT APPLICATION**

(43) Date of publication:
**15.01.2020 Bulletin 2020/03**

(21) Application number: **18305922.9**

(22) Date of filing: **10.07.2018**

(51) Int Cl.:
*B01J 21/18* (2006.01)     *B01J 23/72* (2006.01)
*B01J 23/745* (2006.01)    *B01J 23/78* (2006.01)
*B01J 37/02* (2006.01)     *B01J 37/03* (2006.01)
*B01J 37/16* (2006.01)     *B01J 35/06* (2006.01)
*C07C 29/154* (2006.01)    *C07C 29/156* (2006.01)
*C07C 31/02* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Total Raffinage Chimie**
  **92400 Courbevoie (FR)**
• **ETH Zurich**
  **8092 Zurich (CH)**

(72) Inventors:
• **CURULLA-FERRE, Daniel**
  **1180 UCCLE (BE)**
• **STEWART, Joseph**
  **1180 UCCLE (BE)**
• **PEREZ-RAMIREZ, Javier**
  **8006 ZURICH (CH)**
• **MONDELLI, Cecilia**
  **8057 ZURICH (CH)**
• **LUK, Ho Ting**
  **8049 ZURICH (CH)**

(74) Representative: **Mazurelle, Jean et al
Total Research and Technology Feluy
Zone Industrielle Feluy C
7181 Seneffe (BE)**

(54) **COPPER-IRON-BASED CATALYTIC COMPOSITION FOR THE CONVERSION OF SYNGAS TO HIGHER ALCOHOLS AND PROCESS USING SUCH CATALYST COMPOSITION**

(57)     The invention provides a catalyst composition comprising copper and iron on a support for use in a process for the synthesis of higher alcohols from a syngas feed stream comprising hydrogen and carbon monoxide, wherein the support is a carbon-containing support selected from carbon nanofibers and/or carbon nanotubes, wherein the total content of iron and copper is ranging from 1 to 10 wt% based on the total weight of the catalyst composition and as determined by inductively coupled plasma optical emission spectroscopy, wherein the Cu/Fe ratio is ranging from 0.5:1 to 5:1, and in that the carbon-containing support has a hollow-core structure with an inner diameter of at least 17 nm as determined according to $N_2$ sorption analysis. The invention also provides a process for the synthesis of higher alcohols from a syngas feed stream comprising hydrogen and carbon monoxide using such a catalyst composition.

**EP 3 593 900 A1**

**Description**

Syngas conversion is well known for producing a variety of chemicals including methanol, olefins and paraffin. Such products are produced commercially through the methanol synthesis process or the Fischer-Tropsch synthesis (FTS) process. Higher alcohols ($C_{2+}$) are important compounds with widespread applications in the chemical, pharmaceutical and energy sectors. Currently, they are mainly produced by sugar fermentation (ethanol and isobutanol) or hydration of petroleum-derived alkenes. There is currently no commercially available process for direct synthesis from syngas.

Work on this subject was initiated in the 1930's. However, it has fluctuated with oil prices. In the last decade, interest has increased significantly in line with the rise of shale gas and renewable resources that can generate gaseous feedstocks. No catalytic system reported has performed sufficiently well to justify an industrial implementation to date. The majority of systems that have been explored are generally bulk metal catalysts. They are normally split into four main classes: rhodium-based, molybdenum-based, modified Fischer-Tropsch and modified methanol synthesis catalysts. The majority of catalytic systems contain high loadings of their active metals, which limits their economic viability. A variety of metal combinations including CuCo, CuFe and CoMo as either bulk or supported metals have been described. Bulk materials are generally generated via co-precipitation, while supported materials have been prepared through wet-impregnation, sol-gel and incipient wetness, amongst others. A number of Rh-based systems exist, but their use is limited due to their high price.

Run Xu et al. in "Influence of Promoters on Catalytic Properties of Cu-Mn-Fe/ZrO2 Catalysts for Alcohols Synthesis" React. Kinet. Catal. Lett. 2004, 81, 91-98, found that the addition of iron and manganese has a strong effect on the $Cu/ZrO_2$ catalyst performance for alcohol synthesis. The Cu-Fe species and the interactions between them play an important role in converting the activity centre for methanol synthesis into the centre for higher alcohol synthesis (HAS).

M. Lin et al. In "CO Hydrogenation to Mixed Alcohols over Co-precipitated Cu-Fe Catalysts" Catal. Commun. 2008, 9, 1869-1873 observed that zinc works as an electronic/chemical promoter whereas manganese has a structural role and that the two promoters could act synergistically. They claimed the formation of iron carbides as indispensable for the production of higher alcohols. Hence, a catalyst activated under syngas showed better activity and selectivity to higher alcohols than catalysts activated in $H_2$ or CO.

Mingyue Ding et al. in "Influence of Manganese Promoter on Co-Precipitated Fe-Cu Based Catalysts for Higher Alcohols Synthesis" Fuel 2013, 109, 21-27, found that incorporation of manganese into the Fe-Cu based catalysts facilitated the formation of a Fe-Mn-O solid solution and promoted the dispersion of both iron and copper species. In the HAS reaction, the catalytic activity of CO hydrogenation, the selectivity of $C_{2+}OH$ and hydrocarbons increased gradually with increasing Mn concentration, which may be attributed to the good dispersion of both iron and copper surface active sites in higher manganese content. The optimum conditions for producing higher alcohols from syngas were determined as 270°C and 6.0 MPa.

Kang Xiao et al. in "Structural Evolution of CuFe Bimetallic Nanoparticles for Higher Alcohol Synthesis. "J. Mol. Catal. A: Chem. 2013, 378, 319-325 studied the deactivation process and the structural evolution of CuFe nanoparticles during the HAS reaction process. Alcohol selectivity decreased with time on stream during HAS. This was due to a gradual phase separation during the reaction that isolated Cu from Fe species and weakened the synergism between them.

Zhenghong Bao et al. in "Higher Alcohol Synthesis over Cu-Fe Composite Oxides with High Selectivity to C2+OH" Journal of Energy Chemistry 2013, 22, 107-113, prepared Cu-Fe composites through the co-precipitation method and tested for higher alcohol synthesis from syngas. The selectivity to $C_{2+}OH$ was high whereas the selectivity to methane was low.

Mingyue Ding et al. in "Copper-Iron Supported Bimodal Pore Catalyst and is Application for Higher Alcohol Synthesis" Catalysis Today 2014, 234, 278-284, prepared a Cu-Fe supported bimodal pore catalyst. Starting from the indication that the silica-silica pore support promoted the catalytic activity and selectivity of $C_{2+}OH$, the interaction between the copper and iron species with the bimodal support was further studied. It was found that the increase of active bimetal sites and spatial effect of pore structure probably enhanced the synergistic effect of Cu-Fe, promoting the catalytic activity for HAS.

Xinyou Han et al. in "Effects of Metal Promotion on CuMgFe Catalysts Derived from Layered Double Hydroxides for Higher Alcohol Synthesis via Syngas", RSC Adv. 2015, 5, 51868-51874, prepared Cu-Mg-Fe-M-O (M=Mn, Zr, Ce) catalysts derived from layered double hydroxide (LDHs) precursors, using a co-precipitation method. The catalysts were tested for HAS via carbon. They showed that Mn, Zr and Ce promoters mainly contribute to the formation of octahedrally coordinated copper species which favour the enhancement of the total alcohols selectivity. The addition of Mn facilitates the interaction between Cu and Fe which causes an increase of the total alcohols selectivity and $C_{2+}$ alcohols content. On the contrary, the addition of Zr and Ce promotes the total alcohols selectivity while the $C_{2+}$ alcohols content in the total alcohols decreases.

Xinping Shi et al. in "Synergistic Effect of Nitrogen-doped Carbon-nanotube-supported Cu-Fe Catalyst for the Synthesis of Higher Alcohols from Syngas" Fuel 2017, 210, 241-248 showed that N-doping is an effective approach to improve the catalytic performance of CNT-based catalyst for CO hydrogenation.

There is still a need for a catalyst and a process of HAS with high selectivity to high alcohols and limited $CO_2$ production.

**Summary of the invention**

It is an object of the invention to provide a new process and a new catalyst for HAS from syngas. Another object is to provide a new process and a new catalyst for HAS from syngas allowing improvement in CO conversion to higher alcohols. A further object is to provide a new process and a new catalyst for HAS from syngas allowing improvement in CO conversion together with limited $CO_2$ production. Another object is to provide a new process and a new catalyst for HAS from syngas showing high stability of the catalyst. The present invention provides solution to one or more of the aforementioned needs.

According to a first aspect and to a first definition of the invention, the invention provides a catalyst composition comprising copper and iron on a support for use in a process for the synthesis of higher alcohols from a syngas feed stream comprising hydrogen and carbon monoxide, the catalyst composition being remarkable in that the support is a carbon-containing support selected from carbon nanofibers and/or carbon nanotubes, in that the total content of iron and copper is ranging from 1 to 10 wt% based on the total weight of the catalyst composition and as determined by inductively coupled plasma optical emission spectroscopy, in that the Cu/Fe ratio is ranging from 0.5:1 to 5:1, and in that the carbon-containing support has hollow-core structure with an inner diameter of at least 17 nm as determined according to $N_2$ sorption analysis.

In a preferred embodiment, the carbon-containing support is carbon nanofibers; and/or in that the carbon-containing support has hollow-core structure with an inner diameter of at least 20 nm as determined according to $N_2$ sorption analysis, preferably of at least 25 nm, more preferably of at least 30 nm.

In another preferred embodiment, the composition further comprises at least one promoter selected from alkali and alkaline earth metal.

Surprisingly, it has been found by the Inventors, that improvement in HAS from syngas by CO conversion could be achieved by the combination of a Cu-Fe-based catalyst as active phase, together with a promoter and a carbon-containing support having a hollow-core structure with relatively high inner diameter of at least 17 nm. The catalyst therefore has a corresponding pore diameter of at least 17 nm. The large inner diameter of the hollow-core structure allows to obtain a high HA selectivity of about 30 % as compared to about 20 % for a carbon-containing support having a hollow-core structure of below 16 nm (see example 3).

According to a first aspect and to a second definition of the invention, the invention provides a catalyst composition comprising copper and iron on a support for use in a process for the synthesis of higher alcohols from a syngas feed stream comprising hydrogen and carbon monoxide, the catalyst composition being remarkable in that the support is a carbon-containing support is carbon nanofibers, in that the total content of iron and copper is ranging from 1 to 10 wt% based on the total weight of the catalyst composition and as determined by inductively coupled plasma optical emission spectroscopy, and in that the Cu/Fe ratio is ranging from 0.5:1 to 5:1.

In a preferred embodiment, the carbon-containing support has hollow-core structure with an inner diameter of at least 17 nm as determined according to $N_2$ sorption analysis, preferably at least 20 nm, more preferably of at least 25 nm, and even more preferably of at least 30 nm.

In another preferred embodiment, the composition further comprises at least one promoter selected from alkali and alkaline earth metal.

According to a first aspect and to a third definition of the invention, the invention provides a catalyst composition comprising copper and iron on a support for use in a process for the synthesis of higher alcohols from a syngas feed stream comprising hydrogen and carbon monoxide, the catalyst composition being characterised in that the support is a carbon-containing support, in that the total content of iron and copper is ranging from 1 to 10 wt% based on the total weight of the catalyst composition and as determined by inductively coupled plasma optical emission spectroscopy, in that the Cu/Fe ratio is ranging from 0.5:1 to 5:1, and in that the composition further comprises at least one promoter selected from alkali and alkaline earth metal.

In a preferred embodiment, the carbon-containing support is selected from carbon nanotubes and carbon nanofibers; preferably the carbon-containing support is carbon nanofibers.

In a preferred embodiment, the carbon-containing support has hollow-core structure with an inner diameter of at least 17 nm as determined according to $N_2$ sorption analysis, preferably at least 20 nm, more preferably of at least 25 nm, and even more preferably of at least 30 nm.

Surprisingly, it has been found by the Inventors, that improvement in HAS from syngas by CO conversion could be achieved by the combination of a Cu-Fe-based catalyst as active phase, together with a promoter and a carbon-containing support. Moreover, it was found that it was possible to achieve such improvement with a low metal loading of up to 10 wt% or less than 10 wt% as based on the total weight of the catalyst composition. In addition, the new catalyst shows high stability and low $CO_2$ production.

Indeed, the inventive catalyst composition has been found to be stable, so that the process can be carried out for more than 100 hours, and preferably more than 500 hours, without a significant loss in the catalyst activity.

Moreover, the selectivity to $CO_2$ was kept below 25%.

No matter the definition, with preference one or more of the following features can be used to further define the carbon-containing support:

- The carbon-containing support is selected from carbon nanotubes and carbon nanofiber; preferably the carbon-containing support is carbon nanofibers.
- The carbon-containing support is carbon nanofibers selected from platelet-type carbon nanofibers and conical platelet-type carbon nanofibers; with preference, the carbon-containing support is conical platelet-type carbon nanofibers.
- The carbon-containing support is conical platelet-type carbon nanofibers being iron free, i.e. conical platelet-type carbon nanofibers having a content of iron less than 100 ppm as based on the total weight of the conical platelet-type carbon nanofibers.
- The carbon-containing support has hollow-core structure with an inner diameter of at least 17 nm as determined according to $N_2$ sorption analysis, preferably at least 20 nm, more preferably of at least 25 nm, and even more preferably of at least 30 nm.

Thus, in a preferred embodiment, the catalyst composition comprising copper and iron on a support for use in a process for the synthesis of higher alcohols from a syngas feed stream comprising hydrogen and carbon monoxide, the catalyst composition being remarkable in that the support is carbon nanofibers, in that the total metal loading is ranging from 1 to 10 wt% as based on the total weight of the catalyst composition and as determined by inductively coupled plasma optical emission spectroscopy, in that the Cu/Fe ratio is ranging from 0.5:1 to 5:1, in that the carbon-containing support has hollow-core structure with an inner diameter of at least 17 nm as determined according to $N_2$ sorption analysis, and in that the composition comprises at least one promoter selected from alkali metal and alkaline earth metal.

No matter the carbon-containing support, one or more of the following features can be used to further define the catalyst composition:

- The Cu particle size is at least 7 nm as determined from the (111) reflection in an XRD pattern using the Scherrer equation, preferably at least 8 nm, more preferably at least 9 nm.
- The Cu particle size is at most 35 nm as determined from the (111) reflection in an XRD pattern using the Scherrer equation, preferably at most 30 nm, more preferably at most 25 nm, even more preferably at most 20 nm, most preferably at most 15 nm, and even most preferably at most 11 nm.

The Cu particle size is ranging from 7 to 35 nm as determined from the (111) reflection in an XRD pattern using the Scherrer equation, preferably from 7 to 30 nm, more preferably from 7 to 25 nm, even more preferably from 7 to 20 nm, most preferably from 8 to 15 nm, and even most preferably ranging from 9 to 11 nm.

- The total metal loading is ranging from 2.0 to 8.0 wt% as based on the total weight of the catalyst composition as determined by inductively coupled plasma optical emission spectroscopy, preferably ranging from 3.0 to 7.0 wt%, more preferably from 4.0 to 6.0 wt%; even more preferably from 4.5 to 5.5 wt% and most preferably below 5.0 wt%.
- The Cu/Fe bulk molar ratio is ranging from 1:1 to 4:1, preferably from 1.2:1 to 3:1; more preferably from 1.5:1 to 2.5:1; most preferably the Cu/Fe molar ratio is 2:1.
- At least one promoter is selected from alkali metal, preferably at least one promoter comprises potassium, more preferably at least one promoter is potassium.
- The content of at least one promoter is ranging from 0.001 to 0.5 wt% based on the total weight of the catalyst composition and as determined by inductively coupled plasma optical emission spectroscopy, preferably from 0.001 to 0.4 wt%, more preferably from 0.001 to 0.3 wt%, even more preferably from 0.001 to 0.2 wt%, most preferably from 0.001 to 0.1 wt%, even most preferably from 0.002 to 0.05 wt%, or preferably from 0.003 to 0.03 wt%, or more preferably from 0.004 to 0.02 wt% or more preferably from 0.005 to 0.01 wt% or more preferably from 0.006 to 0.009 wt%.
- The catalyst composition is a reduced catalyst composition.
- The catalyst composition is a reduced catalyst composition as determined by X-ray diffraction wherein the reduced catalyst is devoid of iron oxide.
- The catalyst composition is a reduced composition having a BET surface area in the range of 20 $m^2$ $g^{-1}$ to 300 $m^2$ $g^{-1}$ as determined according to $N_2$ sorption analysis, preferably in the range of 20 $m^2$ $g^{-1}$ to 200 $m^2$ $g^{-1}$.

According to a second aspect, the invention provides a method to produce a catalyst composition according to the first aspect remarkable in that it comprises the following steps:

> i. Co-precipitating iron and copper together, optionally with at least one promoter and re-dissolving them with a complexing agent to form a mixture;
> ii. Depositing the mixture on the carbon-containing support to obtain a slurry;
> iii. Drying the slurry and activating the dried material through reduction to obtain a reduced catalyst composition;
> iv. Optionally calcining the dried catalyst composition to obtain a calcined catalyst composition.

With preference one or more of the following features can be used to further define the inventive method:

- The method is a sol-gel preparation method.
- At least one promoter is an alkali promoter.
- The activation step iii) is performed in diluted $H_2$ at a temperature above 600 K.
- The complexing agent is selected from citric acid, ethylenediaminetetraacetic acid (EDTA), tartaric acid, glycolic acid, oxalic acid, glycine, urea and ethylene glycol; with preference the complexing agent is citric acid.

According to a third aspect, the invention provides a process for the synthesis of higher alcohols from a syngas feed stream comprising hydrogen and carbon monoxide, wherein the process comprises the following steps:

> a) providing a syngas feed stream comprising hydrogen and carbon monoxide;
> b) providing a catalyst composition according to the first aspect;
> c) putting the syngas feed stream in contact with the catalyst composition at a reaction pressure ranging from 1 to 10 MPa and a reaction temperature ranging from 443 K (169.85°C) to 653 K (379.85°C); and
> d) recovering the effluent containing higher alcohols.

With preference one or more of the following features can be used to further define the inventive process:

- The process is carried out in a gaseous phase.
- The step c) is conducted at a *WHSV* of at least 1,000 $cm^3$ $g_{cat}^{-1}$ $h^{-1}$, preferably of at least 2,000 $cm^3$ $g_{cat}^{-1}$ $h^{-1}$, more preferably at least 4,000 $cm^3$ $g_{cat}^{-1}$ $h^{-1}$, even more preferably at least 6,000 $cm^3$ $g_{cat}^{-1}$ $h^{-1}$, and most preferably at least 8,000 $cm^3$ $g_{cat}^{-1}$ $h^{-1}$.
- The syngas feed stream has a molar $H_2$/carbon oxides ratio ranging from 0.5:1 to 12.0:1, preferably ranging from 0.5:1 to 10.0:1, more preferably ranging from 0.5:1 to 8.0:1, even more preferably ranging from 0.5:1 to 6.0:1, most preferably ranging from 0.5:1 to 4.0:1, even most preferably ranging from 0.7:1 to 3.0:1, or preferably ranging from 1.0:1 to 2.5:1, or more preferably ranging from 1.2:1 to 2.2:1; wherein the carbon oxide comprises CO and/or $CO_2$, preferably a mixture of CO and $CO_2$.
- The syngas feed stream has a molar $H_2$/CO ratio ranging from 0.5:1 to 12.0:1, preferably ranging from 0.5:1 to 10.0:1, more preferably ranging from 0.5:1 to 8.0:1, even more preferably ranging from 0.5:1 to 6.0:1, most preferably ranging from 0.5:1 to 4.0:1, even most preferably ranging from 0.7:1 to 3.0:1, or preferably ranging from 1.0:1 to 2.5:1, or more preferably ranging from 1.2:1 to 2.2:1.
- The syngas feed stream comprises at least 5 mol% of CO based on the total molar content of the syngas feed, preferably at least 15 mol%, more preferably at least 17 mol%, more preferably at least 20 mol%.
- The syngas feed stream comprises at least 20 mol% of $H_2$ based on the total molar content of the syngas feed, preferably at least 25 mol%, more preferably at least 27 mol%, more preferably at least 30 mol%.
- The syngas feed stream comprises a mixture of carbon monoxide (CO) and of carbon dioxide ($CO_2$); or the syngas feed stream is devoid of carbon dioxide ($CO_2$).
- The syngas feed stream comprises a mixture of carbon monoxide (CO) and of carbon dioxide ($CO_2$) with the content of $CO_2$ being at most 10 mol% based on the total molar content of the syngas feed, preferably ranging from 0.1 to 10 mol%, more preferably ranging from 0.5 to 8.0 mol%, even more preferably ranging from 1.0 to 6 mol%, and most preferably ranging from 3.0 to 5.0 mol%.
- The pressure is ranging from 2 to 9 MPa, preferably from 3 to 7 MPa, and more preferably from 4 to 6 MPa.
- The temperature is ranging from 493 K (219.85°C) to 553 (279.85°C), preferably from 513 K (239.85°C) to 548 K (274.85°C).
- The process is carried out during more than 100 hours without replacement or reactivation of the catalyst, preferably more than 500 hours.

According to a fourth aspect, the invention provides the use of a Cu-Fe-based catalyst composition in a process for the synthesis of higher alcohols from syngas, the use being remarkable in that the catalyst composition is according to the first aspect.

**Description of the figures**

- Figure 1 shows the $N_2$ sorption isotherms at 77 K of a) activated carbon (AC), b) platelet-type carbon nanofibers ($CNF_p$), c) carbon nanotubes (CNT), and d) conical platelet-type carbon nanofibers (CNF), and of the Cu-Fe catalysts supported on these carriers in reduced and used forms.
- Figure 2 is an XRD diffractogram of the carbonaceous materials tested.
- Figure 3 is a Raman spectrum of the carbonaceous materials tested.
- Figure 4 illustrates the selectivity to product i at a CO conversion level of 7-8% over a) Cu-Fe catalysts in bulk form and supported on different carbonaceous materials, b) Cu-Fe catalysts supported on CNF with variable Cu/Fe molar ratio and prepared by different synthesis methods, and c) Cu-Fe catalysts supported on CNF and promoted with K. Reaction conditions: 543 K, 5 MPa, and molar $H_2/CO$ = 2.
- Figure 5 illustrates the selectivity to product i and the CO conversion over CNF-2/0.005 as a function of a) total pressure, b) temperature, c) $H_2$:CO ratio, d) *WHSV*, and e) Time on Stream (TOS).

**Detailed description of the invention**

As used herein, the terms "catalyst composition" refer to a composition comprising a main active phase on a support, and an alkali promoter. The term catalyst may refer to both a "bulk catalyst" and a "supported catalyst". A bulk catalyst is a catalyst comprising copper and iron. A supported catalyst consists of the catalyst (i.e. the Cu-Fe catalyst) and a support. The metals Cu-Fe are the main active phase, i.e. the active phase, of the supported catalyst.

As used herein the terms "higher alcohols" refer to alcohols containing at least two carbon atoms, such as ethanol, n-propanol; isopropanol; $C_4$ - $C_{20}$ alcohols; *etc.*

In the HAS process according to the invention, a syngas feed stream comprising hydrogen ($H_2$) and carbon oxides (CO alone or a mixture of CO and $CO_2$ gases) is caused to interact with a Cu-Fe-based catalyst composition.

The catalyst composition

The invention contemplates the use of a new catalyst composition in a process for the synthesis of higher alcohols from a syngas feed stream comprising hydrogen and carbon monoxide. In accordance with the invention, the catalyst composition comprises a copper and iron-based catalyst, wherein the Cu-Fe-based catalyst is on a support to form the catalyst composition. The support provides mechanical support to the catalyst as well as further enhancing exposure of the syngas feed stream to the active sites of the catalyst.

According to the invention, the carbon-containing support is preferably selected from carbon nanotubes and carbon nanofiber, more preferably the carbon-containing support comprises carbon nanofibers, even more preferably the carbon-containing support is carbon nanofibers.

When the carbon-containing support is carbon nanofibers, said carbon nanofibers are preferably selected from platelet-type carbon nanofibers and conical platelet-type carbon nanofibers. With preference, the carbon-containing support is conical platelet-type carbon nanofibers.

In a preferred embodiment, the carbon-containing support has hollow-core structure with an inner diameter of at least 17 nm as determined according to $N_2$ sorption analysis, preferably at least 20 nm, more preferably of at least 25 nm, and even more preferably of at least 30 nm.

In a preferred embodiment, the carbon-containing support is conical platelet-type carbon nanofibers being iron free, i.e. conical platelet-type carbon nanofibers having a content of iron less than 100 ppm as based on the total weight of the conical platelet-type carbon nanofibers.

Iron-free conical platelet-type carbon nanofibers are commercially available and are marketed, for example, by Sigma-Aldrich® under SID 329763680.

With preference, the carbon nanofibers have an aspect ratio D x L ranging from 10 nm x 20 $\mu$m to 10 nm x 200 $\mu$m. The average diameter of the carbon nanofibers is ranging from 50 to 200 nm, preferably from 100 to 150 nm. The average pore volume of the carbon nanofibers is ranging from 0.025 $cm^3$/g to 0.125 $cm^3$/g, preferably from 0.05 $cm^3$/g to 0.1 $cm^3$/g. The average pore diameter of the carbon nanofibers is ranging from 50 to 200 Ångström, preferably from 100 to 150 Ångström. The average specific surface area (before deposition of the catalyst and optional calcination) is ranging from 20 to 200 $m^3$/g, preferably from 20 to 50 $m^3$/g.

The total metal loading of the catalyst composition, i.e. the Cu-Fe content, is ranging from 1 to 10 wt% based on the total weight of the catalyst composition and as determined by inductively coupled plasma optical emission spectroscopy. With preference, the total metal loading is ranging from 2.0 to 8.0 wt% based on the total weight of the catalyst composition as determined by inductively coupled plasma optical emission spectroscopy, preferably ranging from 3.0 to 7.0 wt%, more preferably from 4.0 to 6.0 wt%; even more preferably from 4.5 to 5.5 wt% and most preferably below 5.0 wt%. In an embodiment, the total metal loading is ranging from 2.0 to 4.9 wt% based on the total weight of the catalyst composition as determined by inductively coupled plasma optical emission spectroscopy.

The Cu/Fe bulk molar ratio of the catalyst composition is ranging from 0.5:1 to 5:1. With preference, the Cu/Fe molar ratio is ranging from 1:1 to 4:1, preferably from 1.2:1 to 3:1; more preferably from 1.5:1 to 2.5:1; most preferably the Cu/Fe molar ratio is 2:1.

In a preferred embodiment, the Cu particle size is ranging from 7 to 35 nm as determined from the (111) reflection in an XRD pattern using the Scherrer equation, preferably from 7 to 30 nm, more preferably from 7 to 25 nm, even more preferably from 7 to 20 nm, most preferably from 8 to 15 nm, and even most ranging from 9 to 11 nm.

In a preferred embodiment, the catalyst composition further comprises at least one promoter selected from alkali and alkaline earth metal. With preference, at least one promoter is selected from alkali metal, preferably at least one promoter comprises potassium, more preferably at least one promoter is potassium.

In a preferred embodiment, the content of the at least one promoter is ranging from 0.001 to 0.5 wt% based on the total weight of the catalyst composition and as determined by inductively coupled plasma optical emission spectroscopy, preferably from 0.001 to 0.4 wt%, more preferably from 0.001 to 0.3 wt%, even more preferably from 0.001 to 0.2 wt%, most preferably from 0.001 to 0.1 wt%, even most preferably from 0.002 to 0.05 wt%, or preferably from 0.004 to 0.03 wt%, or more preferably from 0.006 to 0.02 wt%.

The catalyst composition is a calcined catalyst composition having a BET surface area in the range of about 20 $m^2$ $g^{-1}$ to 400 $m^2$ $g^{-1}$ as determined according to $N_2$ sorption analysis.

With preference, the catalyst composition is a reduced catalyst composition as determined by X-ray diffraction wherein the reduced catalyst is devoid of iron oxide.

Method to prepare the catalyst composition

The invention also provides a method to produce a catalyst composition according to the first aspect that is remarkable in that it comprises the following steps:

 i. Co-precipitating iron and copper together, optionally with at least one promoter, and re-dissolving them with a complexing agent to form a mixture;
 ii. Depositing the mixture on the carbon-containing support to obtain a slurry;
 iii. Drying the slurry and activating the dried material through reduction to obtain a reduced catalyst composition;
 iv. Optionally calcining the dried catalyst composition to obtain a calcined catalyst composition.

As it is clear to the person skilled in the art, the method to produce a catalyst composition according to the first aspect is preferably a sol-gel method. With preference, at least one promoter is an alkali promoter.

The complexing agent is selected from citric acid, ethylenediaminetetraacetic acid (EDTA), tartaric acid, glycolic acid, oxalic acid, glycine, urea and ethylene glycol; with preference the complexing agent is citric acid.

With preference, step i) of co-precipitating iron and copper together with the promoter to form a mixture comprises the following sub-steps:

-  Preparing a solution of copper (II) nitrate hydrate and iron (III) nitrate hydrate with water;
-  Adding $NH_4OH$ to the solution to form a precipitate and dissolving the recovered solid in a complexing agent solution, preferably a citric acid solution;
-  Adding a desired amount of an alkali carbonate, preferably potassium carbonate.
-  Optionnaly, adjusting the pH of the mixture to value preferably between 2 and 5, more preferably between 3 and 5, and most preferably between 3 and 4.

With preference, the activation step iii) is performed in diluted $H_2$ at a temperature above 600 K.

The HAS process

The invention provides a process for the synthesis of higher alcohols from a syngas feed stream comprising hydrogen and carbon monoxide, wherein the process comprises the following steps:

 a) providing a syngas feed stream comprising hydrogen and carbon monoxide;

b) providing a catalyst composition as described above;
c) putting the syngas feed stream in contact with the catalyst composition at a reaction pressure ranging from 1 to 10 MPa and a reaction temperature ranging from 443 K (169.85°C) to 653 K (379.85°C); and
d) recovering the effluent containing higher alcohols.

The process can be carried out in gaseous phase or in liquid phase. The solvent that can be used for the reaction in liquid phase includes hydrocarbons and other solvents which are preferably insoluble or sparingly soluble in water. The process can be carried out in liquid phase or in gaseous phase. Preferably, the process is carried out in gaseous phase.

The process is carried out in a reactor comprising:

- lines to introduce a syngas as feed stream to the reactor and to remove product from the reactor;
- a device for heating the reactor;
- a temperature sensor and controller to control the reactor temperature to be within the reaction temperature range of 443 K (169.85°C) to 653 K (379.85°C);
- flow controllers to control the rate of the feed stream to the reactor; and
- a pressure controller to control the reactor pressure to be within the reaction pressure range of from 1 to 10 MPa.

In accordance with the invention, the syngas feed stream comprises hydrogen ($H_2$) and carbon oxides (CO alone or a mixture of CO and $CO_2$ gases). With preference, the feed stream comprises hydrogen ($H_2$) and carbon monoxide (CO).

In a preferred embodiment, the syngas feed stream comprises at least 20 mol% of hydrogen ($H_2$) based on the total molar content of the syngas feed, preferably at least 25 mol%, more preferably at least 27 mol%, and more preferably at least 30 mol%.

In an embodiment, the syngas feed stream comprises at most 90 mol% of hydrogen ($H_2$) based on the total molar content of the syngas feed, preferably at most 80 mol%, more preferably at most 70 mol%, and even more preferably at most 60 mol%.

In a preferred embodiment, the syngas feed stream comprises at least 10 mol% of carbon monoxide (CO) based on the total molar content of the syngas feed, preferably at least 15 mol%, more preferably at least 17 mol%, and more preferably at least 20 mol%.

In an embodiment, the syngas feed stream comprises at most 90 mol% of carbon monoxide (CO) based on the total molar content of the syngas feed, preferably at most 80 mol%, more preferably at most 70 mol%, and more preferably at most 60 mol%.

In a preferred embodiment, the syngas feed stream has a molar $H_2$/carbon oxides ratio ranging from 0.5:1 to 12.0:1, preferably ranging from 0.5:1 to 10.0:1, more preferably ranging from 0.5:1 to 8.0:1, even more preferably ranging from 0.5:1 to 6.0:1, most preferably ranging from 0.5:1 to 4.0:1, even most preferably ranging from 0.7:1 to 3.0:1, or preferably ranging from 1.0:1 to 2.5:1, or more preferably ranging from 1.2:1 to 2.2:1; wherein the carbon oxide comprises CO and/or $CO_2$, preferably a mixture of CO and $CO_2$.

The syngas feed stream has a molar $H_2$/CO ratio ranging from 0.5:1 to 12.0:1, preferably ranging from 0.5:1 to 10.0:1, more preferably ranging from 0.5:1 to 8.0:1, even more preferably ranging from 0.5:1 to 6.0:1, most preferably ranging from 0.5:1 to 4.0:1, even most preferably ranging from 0.7:1 to 3.0:1, or preferably ranging from 1.0:1 to 2.5:1, or more preferably ranging from 1.2:1 to 2.2:1.

The syngas feed stream used in the process of the invention comprises CO and $H_2$, or $H_2$ and a mixture of CO and $CO_2$. Preferably, the syngas feed stream may also comprise a further gaseous component such as an inert gas. The inert gas is for example argon.

In an embodiment, the syngas feed stream comprises a mixture of carbon monoxide (CO) and of carbon dioxide ($CO_2$) with the content of $CO_2$ being at most 10 wt% based on the total molar content of the syngas feed, preferably ranging from 0.1 to 10 wt%, more preferably ranging from 0.5 to 8.0 wt%, even more preferably ranging from 1.0 to 6 wt%, and most preferably ranging from 3.0 to 5.0 wt%.

The process is carried at a reaction temperature ranging from 443 K (169.85°C) to 653 K (379.85°C), and preferably from 493 K (219.85°C) to 553 K (279.85°C). The reaction temperature is preferably at least 498 K (224.85°C), more preferably at least 503 K (229.85°C), even more preferably at least 508 K (234.85°C) and most preferably at least 513 K (239.85°C); and/or the reaction temperature is preferably at most 548 K (274.85°C). The person skilled in the art may increase the reaction temperature in order to increase the conversion rate of the carbon monoxide.

The higher alcohols are preferably recovered in gaseous phase, and further subjected to a separation treatment. The process is carried at a reaction pressure ranging from 1.0 to 10.0 MPa, preferably ranging from 2.0 to 9.0 MPa, more preferably ranging from 3.0 to 7.0 MPa. The reaction pressure is preferably at least 3.5 MPa, more preferably at least 4.0 MPa; and/or, the reaction pressure is preferably at most 6.5 MPa, more preferably at most 6.0 MPa.

In a preferred embodiment, the step c) of putting the syngas feed stream in contact with the catalyst composition, is conducted at a weight hourly space velocity (*WHSV*) of at least 1,000 cm$^3$ g$_{cat}^{-1}$ h$^{-1}$ at standard temperature and pressure (STP). The weight hourly space velocity is defined in volume of reactant gases per hour per weight of catalyst composition charged to the reactor. With preference, the weight hourly space velocity (*WHSV*) is of at least 2,000 cm$^3$ g$_{cat}^{-1}$ h$^{-1}$, preferably at least 4,000 cm$^3$ g$_{cat}^{-1}$ h$^{-1}$, more preferably at least 6,000 cm$^3$ g$_{cat}^{-1}$ h$^{-1}$, even more preferably at least 8,000 cm$^3$ g$_{cat}^{-1}$ h$^{-1}$; and/or of at most 100,000 cm$^3$ g$_{cat}^{-1}$ h$^{-1}$, preferably at most 50,000 cm$^3$ g$_{cat}^{-1}$ h$^{-1}$, more preferably at most 40,000 cm$^3$ g$_{cat}^{-1}$ h$^{-1}$, even more preferably at most 30,000 cm$^3$ g$_{cat}^{-1}$ h$^{-1}$.

According to the invention, the process can be carried out with a stable performance with respect to activity and selectivity during more than 100 hours without replacement or reactivation of the catalyst.

In an embodiment, the process is carried out in a fixed bed or fluidised bed reactor comprising at least one catalytic bed. Such reactors are well-known from the person skilled in the art and for instance described in EP2257366 or in US7279138.

**Tests methods and definitions**

The K. Cu. and Fe contents were determined by inductively coupled plasma optical emission spectroscopy (ICP-OES) using a Horiba Ultra 2 instrument equipped with a photomultiplier tube detector. For the determination of the Cu and Fe content, 5 mg of catalyst were suspended in 0.5 cm$^3$ of concentrated nitric acid (65%, as received), kept in an ultrasonic bath at 323 K for 3 h, diluted with deionised water to a final volume of 10 cm$^3$, and filtered prior to measurement. Concentrated nitric acid was chosen because metallic and oxidic Cu and Fe (Cu, Cu$_2$O, CuO, Fe$_3$O$_4$, and Fe$_2$O$_3$) were proved to dissolve completely. For the determination of the K content in samples with nominal K/total metals ratio of 0.005, 0.01, and 0.1, the same procedure was followed but catalyst amounts of 100, 60, and 5 mg, respectively, were used.

X-ray fluorescence spectroscopy (XRF) was performed using an Orbis Micro-EDXRF spectrometer equipped with an Rh source operated at 35 kV and 500 μA and a silicon drift detector to obtain the bulk Cu/Fe molar ratio in CP-2.

N$_2$ sorption at 77 K was measured in a Micromeritics TriStar II instrument after degassing the samples at 573 K under vacuum for 3 h.

The surface area of catalysts and supports was determined by applying the BET method.

Powder X-ray diffraction (XRD) was conducted using a PANalytical X'Pert Pro-MPD diffractometer with Ni-filtered Cu K*a* radiation (*l* = 0.1541 nm), acquiring data in the 10-70° 2$\theta$ range with an angular step size of 0.033° and a counting time of 8 s per step.

The size of metallic copper and iron crystallites was determined using the Scherrer equation or by microscopy.

The size and location of metallic copper and iron particles were determined by high-resolution transmission electron microscopy (HRTEM) and scanning transmission electron microscopy coupled to energy dispersive X-ray spectroscopy (STEM-EDX), which were conducted in an FEI Talos F200A instrument equipped with a high brightness field emission gun, a high-angle annular dark-field (HAADF) and a large collection angle EDX detector operated at 200 kV. Catalyst powders were dispersed on nickel grids coated with a continuous carbon film.

(High-pressure) temperature-programmed desorption of H$_2$ ((HP-)H$_2$-TPD) was carried out using a Micromeritics Autochem 2950 HP unit equipped with a thermal conductivity detector and coupled to a Pfeiffer Vacuum Omnistar™ GSD-320 quadrupole mass spectrometer. Reduced samples (0.250 g) were treated in a 10 vol.% H$_2$/N$_2$ flow of 10 cm$^3$ min$^{-1}$ at 0.5 MPa and 573 K for 3 h (3 K min$^{-1}$) to simulate the activation. After cooling down to the reaction temperature (543 K), the reducing gas was replaced by 60 vol.% H$_2$/He, followed by He purging at a flow of 20 cm$^3$ min$^{-1}$ for 1.5 h. H$_2$-TPD curves were acquired while heating up to 1073 K (5 K min$^{-1}$) in the same He flow. For the high-pressure analysis, the pressure was increased to 5 MPa when admitting 60 vol.% H$_2$/He after sample reduction and cooling. The H$_2$ adsorption and He purging times were equal to the ambient-pressure experiments.

X-ray photoelectron spectroscopy (XPS) was conducted using a Physical Electronics (PHI) Quantum 2000 X-ray photoelectron spectrometer featuring monochromatic Al Ka radiation, generated from an electron beam operated at 15 kV and 32.3 W, and a hemispherical capacitor electron-energy analyser, equipped with a channel plate and a position-sensitive detector. The energy scale of the instrument was calibrated using Au and Cu as reference samples. The catalysts were firmly pressed onto indium foil patches, which were then mounted onto a sample plate and introduced into the spectrometer. The analysis was conducted at $1\times10^{-6}$ Pa, with an electron take-off angle of 45° and a pass energy of 46.95 eV. Charge compensation during the measurement was achieved using a low energy electron source. Surface elemental concentrations were determined from the photoelectron spectra after Shirley background subtraction using the instrument specific sensitivity factors for calculation. The measurements were repeated after conducting sputtering with Ar$^+$ ions at 1 kV for 0.5 min over an area of approximately 2 $\times$ 2 mm$^2$.

Raman spectroscopy was used to determine the nature of the carbon support, specifically the identification of the stretching of bonds between sp$^2$-hybridized C atoms in graphite and the stretching of bonds of the same atoms at defect positions (distorted atoms at edges or in the lattice). The analysis was carried out using a confocal Raman microscope (WITec CRM 200) equipped with a 532-nm diode laser and a 100x objective lens, operated in the backscattering mode with a power of 6 mW.

## Examples

The advantages of the present invention are illustrated by the following examples. However, it is understood that the invention is not limited to these specific examples.

### Example 1: Catalysts preparation

#### Bulk catalyst

A bulk Cu-Fe catalyst with nominal molar Cu/Fe = 2 was prepared by co-precipitation using $Na_2CO_3$ as the precipitating agent. $Cu(NO_3)_2 \cdot 3H_2O$ (5.283 g, Aldrich Fine Chemicals, 98-103%) and $Fe(NO_3)_3 \cdot 9H_2O$ (4.406 g, Aldrich Fine Chemicals, >98%) were dissolved simultaneously in deionised water (65.6 $cm^3$) yielding a solution with a total metals concentration of 0.5 M. The mixture was magnetically stirred at 343 K while adding a 0.5 M $Na_2CO_3$ solution (81-83 $cm^3$, 2 $cm^3$ $min^{-1}$) to reach a pH of 8. The slurry obtained was aged for 3 h, washed with water (3 $dm^3$), dried at 343 K, calcined at 723 K, and reduced in a 10 vol.% $H_2$/He flow of 20 $cm^3$ $min^{-1}$ for 4 h at 673 K (ramp rate = 3 K $min^{-1}$).

#### Supported catalysts

Supported Cu-Fe catalysts with nominal molar Cu/Fe molar ratios of 1, 2, 3, or 5, and a Cu-Fe loading of 5 wt% were prepared by a sol-gel method (SG). K was added as a promoter to the materials with Cu/Fe = 2 in a nominal molar K/Cu-Fe molar ratio of 0.005, 0.01, or 0.1. Conical platelet-type CNF (Aldrich-Fine Chemicals, Fe content < 100 ppm, denoted as CNF), platelet-type CNF (ABCR, >98%, denoted as $CNF_p$), multi-walled carbon nanotubes (ABCR, 95%, outer diameter = 20-30 nm, denoted as CNT), and activated carbon (ACROS Organics Co., Ltd., denoted as AC) were used as carriers as received.

$Cu(NO_3)_2 \cdot 3H_2O$ (2.130-3.404 g) and $Fe(NO_3)_3 \cdot 9H_2O$ (1.138-3.562 g) were dissolved in deionised water (11.5 $cm^3$) under magnetic stirring. With the addition of 4-5 drops of $NH_4OH$ (Acros Organics, 25 wt% aqueous solution), a dark green precipitate was formed. A citric acid (Sigma-Aldrich, ≥95%) solution (1.30-1.36 g in 3 $cm^3$ of water, corresponding to a molar citric acid/Cu-Fe ratio of 0.4) was then added under magnetic stirring to re-dissolve the solid. When desired, an adequate amount of $K_2CO_3$ solution (0.006-0.12 g in 1 $cm^3$ of water) was incorporated. The pH of the solution was adjusted to 3.5 adding few drops of formic acid (Merck product, 98-100%) and $NH_4OH$. An aliquot of this final mixture was added to 2.0 g of support to achieve the desired metals loading. The slurry was magnetically stirred for 6 h at 338 K and dried in air at the same temperature overnight. The obtained solid was sieved to attain a 0.05-0.12 mm fraction, which was activated by reduction in a 10 vol.% $H_2$/He flow of 20 $cm^3$ $min^{-1}$ for 4 h at 673 K (3 K $min^{-1}$).

These catalysts were coded combining information on the support (abbreviation), the molar Cu/Fe ratio, and the molar relative amount of K in the catalyst (if relevant), e.g., CNF-2-0.01. A portion of the CNF-2 catalyst was calcined in air for 3 h at 573 K (5 K $min^{-1}$) prior to the reduction step (denoted as CNF-2-C).

The sol-gel method was applied to prepare additional catalysts supported on CNF with a nominal Cu/Fe = 2 through sequential addition of the metals, *i.e.,* Fe/Cu/CNF-2 (Cu incorporated prior to Fe), K/Cu-Fe/CNF-2-0.005 (K added after the simultaneous deposition of Cu and Fe), Fe/Cu-K/CNF-2-0.005 (Fe added after the simultaneous incorporation of Cu and K), and Cu-Fe/K/CNF-2-0.005 (Cu and Fe added simultaneously after K deposition). The amount of citric acid used during the individual deposition of Cu or Fe was adjusted to attain the same molar ratio as upon the one-pot deposition of the two metals. The solids were reduced after each incorporation of Cu and/or Fe applying the conditions detailed above.

Supported catalysts with nominal molar Cu/Fe = 2 and Cu-Fe loading of 5 wt.% and CNF as the carrier were alternatively prepared by a wet deposition-reduction approach using $NaBH_4$ as the reducing agent (denoted as CR-CNF-2) and by deposition-precipitation using $Na_2CO_3$ as the precipitating agent (denoted as DP-CNF-2). Along the former route, $Cu(NO_3)_2 \cdot 3H_2O$ (0.2773 g) and $Fe(NO_3)_3 \cdot 9H_2O$ (0.2318 g) were dissolved in deionised water (172 $cm^3$) to attain a total concentration of metals of 0.01 M. 2 g of CNF were added to this aqueous solution and the mixture was magnetically stirred at 298 K for 1 h. Afterwards, a 0.5 M $NaBH_4$ solution was introduced (51 $cm^3$, 2 $cm^3$ $min^{-1}$) and the mixture was kept under stirring overnight at 298 K. The solid was recovered by filtration, washed with water (3 $dm^3$), and dried in vacuum overnight at 343 K. DP-CNF-2 was prepared by dissolving the same amounts of salts of metals in 86 $cm^3$ of water, leading to a total concentration of metals of 0.02 M. 2 g of CNF were introduced to this solution and the mixture was refluxed under stirring at 333 K for 1 h. Thereafter, a 0.04 M $Na_2CO_3$ solution was added (55 $cm^3$, 2 $cm^3$ $min^{-1}$) to the mixture until a pH of 8 was attained. The slurry was aged at the same temperature for 3 h. The solid was recovered by filtration, washed with water (3 $dm^3$), dried overnight in air at 333 K, calcined in air for 4 h at 723 K (5

K min$^{-1}$), and finally reduced under the aforementioned conditions.

**Example 2: Catalyst testing in HAS process**

The direct conversion of syngas to higher alcohols was carried out in a continuous-flow fixed-bed reactor setup, consisting of (*i*) mass flow controllers to feed H$_2$, CO and Ar (Messer, $\geq$ 99.997%) and a liquid injection system based on a controlled evaporator unit (Bronkhorst) to feed alcohols and hydrocarbons for calibration, (*ii*) a nickel-lean steel reactor (0.65 cm i.d.) equipped with a thermocouple placed directly in the catalytic bed and heated by an oven, (iii) a hot trap kept at 453 K to condense alcoholic and aliphatic products with more than 6 and 10 carbon atoms, respectively, and (*iv*) an online GC (Agilent 6890A) comprising two columns (ShinCarbon ST and PoraPLOT Q PT), a thermal conductivity detector (TCD) and a flame ionisation detector (FID) to monitor the outlet gas composition. 0.5 g (sieve fraction = 0.05-0.12 mm) of undiluted catalyst were loaded into the reactor and purged with an Ar (Messer, purity 5.0) flow of 100 cm$^3$ min$^{-1}$ for 0.5 hour at ambient pressure. Under the same flow, the pressure was then increased to 5 MPa and a leak test was conducted. Thereafter, the catalyst was activated by flowing 10 vol% H$_2$/Ar at the same rate as for the inert gas previously used: 0.5 MPa and 573 K (3 K min$^{-1}$) for 3 h. The reaction was carried out by feeding a mixture of H$_2$ (Messer, purity 5.0), CO (Messer, purity 5.0), and Ar with a molar H$_2$/CO/Ar ratio of 6/3/1 at 543 K, 5 MPa, and a *WHSV* of 4000 cm$^3$ g$_{cat}$$^{-1}$ h$^{-1}$. In order to compare different catalysts at the same CO conversion level (7-8%), the feed flow rate was varied (*WHSV* = 500-48000 cm$^3$ g$_{cat}$$^{-1}$ h$^{-1}$). The data relative to this comparison represents the average value of 4-5 measurements taken between ca. 11-15 h on stream. Kinetic investigations were additionally conducted over the best catalyst by varying the temperature, pressure, H$_2$/CO ratio, and *WHSV* between 473-593 K, 2-7 MPa, 0.2-4, and 500-32000 cm$^3$ g$_{cat}$$^{-1}$ h$^{-1}$, respectively. The results reported were determined averaging values obtained from 4-5 measurements between ca. 6-10 h on stream. The best catalyst was also assessed in a 100-h test. In this case the data refers to a single measurement. The CO conversion ($x_{CO}$) was calculated using equation (1):

$$X_{CO} = \left[ \frac{\dot{n}_{CO,in} - \dot{n}_{CO,out}}{\dot{n}_{CO,in}} \right] x100 \qquad (1)$$

where $\dot{n}_{CO,in}$ and $\dot{n}_{CO,out}$ are the molar flows of CO (expressed in mmol h$^{-1}$) at the inlet and outlet of the reactor, respectively. The selectivity to product $i(S_i)$ was calculated using equation (2):

$$S_i = \frac{\dot{n}_{i,out} N_{c,i}}{\sum \dot{n}_{i,out} N_{c,i}} x100 \qquad (2)$$

where $\dot{n}_{i,out}$ and $N_{c,i}$ are the molar flow of product *i* and the number of carbon atoms in product *i*, respectively. The selectivity to HA was obtained summing the individual selectivities to alcohols with 2 or more carbon atoms, while the selectivity to HC summing the individual selectivities to HC with 1 or more carbon atoms. The space-time yield of HA ($STY_{HA}$) expressed in g$_{HA}$g$_{cat}$$^{-1}$h$^{-1}$ was calculated using equations (3):

$$STY_{HA} = \sum S_{j,HA} \, MW_{j,HA} \frac{X_{CO} \dot{n}_{CO,in}}{m_{cat}} \qquad (3)$$

where $m_{cat}$ is the mass of the catalyst and $MW_j$,HA is the molecular weight of (higher) alcohols containing *j* carbon atoms. The carbon balance was determined according to equation (4) and was always higher than 95%.

$$\varepsilon_C = \frac{\dot{n}_{CO,in} - \sum \dot{n}_{i,out} N_{c,i}}{\dot{n}_{CO,in}} \times 100\% \qquad (4)$$

**Example 3: Effect of the structure of the carbon carriers**

Four carbonaceous materials were tested as support for the catalysts:

- activated carbon (AC);
- platelet-type carbon nanofibers ($CNF_p$);
- carbon nanotubes (CNT); and
- conical platelet-type carbon nanofibers (CNF).

AC and $CNF_p$ possess high surface areas. CNF and CNT have a hollow-core structure which offers the possibility to limit the growth of particles of metals due to confinement effects.

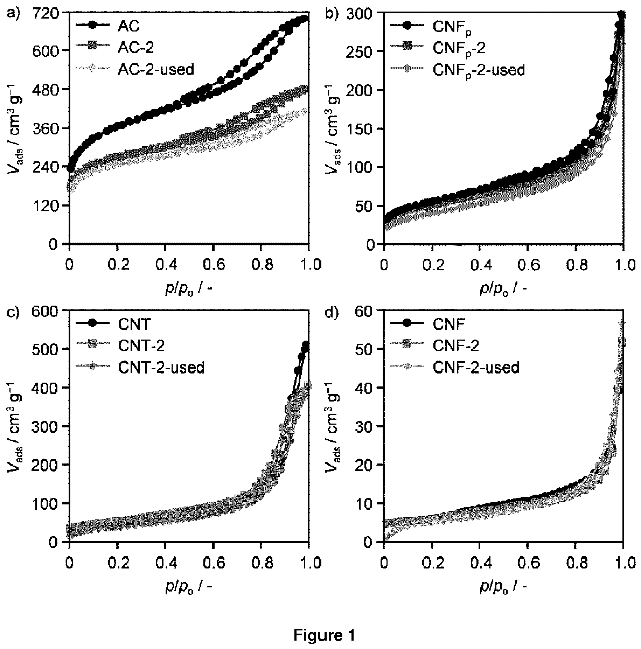

The porous, structural and morphological properties of the four carbon carriers were assessed by $N_2$ sorption (see **Table 1** and **Figure 1**), XRD, transmission electron microscopy (TEM) and Raman spectroscopy. Surface areas and pore volumes were comparable to literature data for all materials.

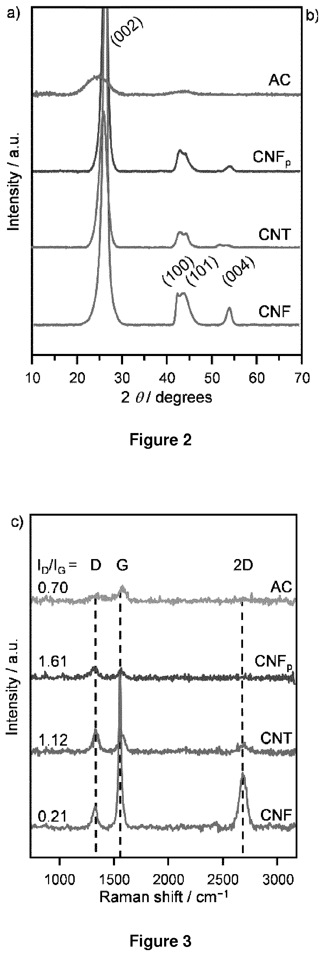

The surface area was the largest for AC, which was followed by $CNF_p$, CNT, and finally CNF. The diffractogram (see **Figure 2**) of AC evidenced two very broad peaks, highlighting the amorphous nature of this sample, while the patterns of CNF, $CNF_p$, and CNT featured reflections specific to graphitic carbon at $2\theta = 24.4, 42.4, 43.8,$ and $54.0°$, corresponding to the (002), (100), (101), and (004) planes, respectively. The diffraction peak of the (002) plane is broader and slightly shifted towards lower angle for CNT, indicating a larger $d_{002}$ spacing.

The Raman spectra of the four supports are given in **Figure 3** and evidenced two characteristic bands at about 1580 and 1330 $cm^{-1}$. The former is attributed to the stretching of the bonds between the $sp^2$-hybridized C atoms in graphite (G-band), while the latter to the stretching of the bonds of the same atoms at defect positions (distorted atoms at edges or in the lattice, D-band). The ratio of the intensity of the D- and G-bands ($I_D/I_G$), which is a measure of the density of defect sites, increased in the order CNF (0.21) < AC (0.70) < CNT (1.12) < $CNF_p$ (1.61), in line with the literature. The high defect concentration in $CNF_p$ originates from the small size of the graphene sheets forming its structure and the consequent exposure of a higher fraction of edges. In contrast, CNT comprises long sheets rolled up in tubes, exposing defects only at their openings. The amount of defects in CNF is even lower due to the high order in the stacking of the individual graphene layers. The ordered carbon structure of CNF and CNT is further corroborated by the presence of the overtone band at about 2700 $cm^{-1}$ (2D-band).

Cu-Fe catalysts supported on these carbonaceous materials with nominal metals loading of 5 wt% and molar Cu/Fe ratio of 2 were prepared by a sol-gel method and activated by reduction in diluted $H_2$ at 673 K.

A bulk Cu-Fe catalyst with a molar Cu/Fe ratio of 2 was prepared by co-precipitation, calcined in air at 723 K for 3 h, and activated under the same conditions as above to serve as a reference.

**Table** 1. Characterisation data of the supported CuFe catalysts and a bulk CuFe catalyst in reduced forms.

| Catalysts | Bulk Cu-Fe content[a] [wt%] | Bulk molar Cu/Fe ratio[a] | Surface molar Cu/Fe ratio[b] | $S_{BET, reduced}$[c,d] [$m^2g^{-1}$] | $S_{BET,used}$[d] [$m^2g^{-1}$] | $V_{pore, reduced}$[c,e] [$cm^3g^{-1}$] | $V_{pore, used}$[e] [$cm^3g^{-1}$] | $d_{Cu}$[f] [nm] |
|---|---|---|---|---|---|---|---|---|
| AC-2 | 5.2 | 2.05/1 | - | 866 (1154) | 792 | 0.74 (1.05) | 0.64 | 23.9 (22.4) |
| $CNF_p$-2 | 4.8 | 2.11/1 | - | 184 (201) | 151 | 0.43 (0.43) | 0.37 | 13.1 (25.0) |
| CNT-2 | 4.9 | 1.93/1 | - | 190 (168) | 142 | 0.60 (0.77) | 0.58 | 7.2 (9.7) |
| CNF-2 | 4.9 | 1.93/1 | 0. 8 | 28 (28) | 19 | 0.08 (0.08) | 0.06 | 9.5 (9.9) |
| CP-2 | - | 1.94/1 | - | 6 (63) | 3 | 0.02 (0.16) | 0.01 | 37.8 (38.8) |

[a] ICP-OES, XRF for CP-2.

[b] XPS.

[c] Data for carriers and CP-2 in the calcined form in brackets.

[d] BET method.

[e] t-plot method.

[f] Determined from the (111) reflection in the XRD pattern using the Scherrer equation. Data for used samples in brackets.

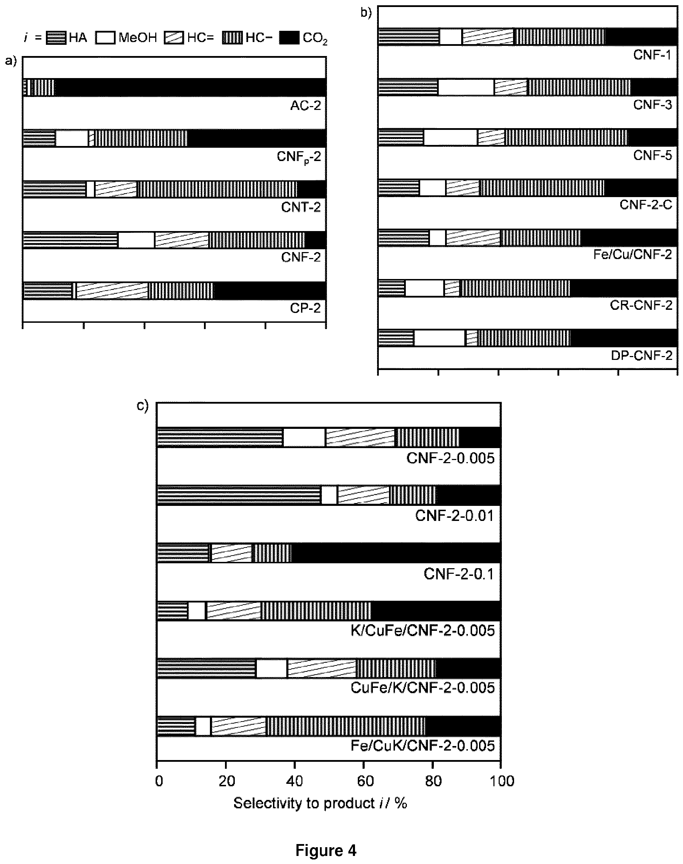

The performance of these catalytic systems in the direct conversion of syngas to HA was investigated at a temperature of 543 K, a total pressure of 5 MPa and a molar $H_2/CO$ ratio of 2 (see **Figure 4a** and **Table 2**). The supported catalysts exhibited a very different activity upon an initial testing at *WHSV* of 4000 $cm^3$ $g_{cat}^{-1}$ $h^{-1}$, with AC-2 and $CNF_p$-2 being almost an order of magnitude less active than the other two systems. Accordingly, the *WHSV* was varied between 500-32000 $cm^3$ $g_{cat}^{-1}$ $h^{-1}$ to compare their selectivity at the same CO conversion ($X_{CO}$ = 7-8%). A very diverse product distribution was observed.

Table 2: Performance data for the catalysts investigated.

| Catalyst[a] | *WHSV* | $STY_{HA[ROH]}$[a] | $X_{CO}$ | $S_{MeOH}$ | $S_{HA}$ | $S_{HC=}$ | $S_{HC-}$ | $S_{CO2}$ |
|---|---|---|---|---|---|---|---|---|
| | ($cm^3$ $g_{cat}^{-1}$ $h^{-1}$) | ($g_{HA}$ $g_{cat}^{-1}$ $h^{-1}$) | (%) | (%) | (%) | (%) | (%) | (%) |
| CNF-2 | 4,000 | 0.13 [0.19] | 48 | 7 | 20 | 7 | 49 | 17 |
| | 28,000 | 0.23 [0.37] | 8 | 13 | 31 | 18 | 32 | 6 |
| $CNF_p$-2 | 4,000 | 0.01 [0.03] | 8 | 11 | 11 | 2 | 31 | 45 |
| | 12,000 | 0.05 [0.08] | 6 | 9 | 17 | 8 | 30 | 36 |
| CNT-2 | 4,000 | 0.07 [0.10] | 35 | 5 | 15 | 2 | 62 | 16 |
| | 12,000 | 0.06 [0.08] | 7 | 3 | 21 | 14 | 53 | 9 |
| AC-2 | 4,000 | n.r. | 3 | 1 | 0 | 1 | 3 | 95 |
| | 500 | n.r. | 8 | 2 | 2 | 1 | 7 | 88 |
| CP-2 | 4,000 | 0.15 [0.16] | 95 | 0.5 | 12 | 4 | 53 | 31 |
| | 48,000 | 0.19 [0.21] | 7 | 1 | 16 | 24 | 22 | 36 |
| [a] Reaction conditions: 543 K, 5 MPa and molar $H_2/CO$ = 2. HA = higher alcohols, MeOH = methanol, HC= = olefins and HC- = paraffins. n.r. = not relevant, because negligible. | | | | | | | | |

From the results, it can be seen that CNF-2 achieved the highest HA selectivity (31%) and was followed by CNT-2 (21%), $CNF_p$-2 (11%), and AC-2 (2%). $CO_2$ production vastly deviated from one system to another. AC-2 and $CNF_p$-2 displayed an extremely high $CO_2$ selectivity (88 and 45%, respectively). In stark contrast, the formation of this much-undesired product was very limited over CNT-2 and CNF-2 (9 and 6%, respectively).

CP-2 exhibited a very high CO conversion under standard conditions (95%), but at a rather low HA selectivity (12%). The HA selectivity was slightly higher (16%) at the same activity level as for the supported systems, but the catalyst still mainly produced hydrocarbons (46% in total with about a 1:1 ratio among alkenes and alkanes) and $CO_2$ (36%).

### Example 4: Effect of the Cu particle size

Based on the Scherrer equation, the Cu particle size followed the trend CP-2 (37.8 nm) > AC-2 (23.9 nm) > $CNF_p$-2 (13.1 nm) > CNF-2 (9.5 nm) > CNT-2 (7.2 nm) (see **Table** 1).

Reflections specific to metallic Fe ($2\theta$ = 44.8 and 65.2°) were identified in the diffractograms of CNF-2, CNT-2, and $CNF_p$-2, and indicated that Fe was more finely dispersed on CNF and CNT. CP-2 comprised $Fe_3O_4$ (32.8 nm) as the only Fe-containing phase.

Since the metal particles are predominantly located in the hollow cores in the CNT- and CNF-supported catalysts, their smaller size compared to the AC- and $CNF_p$-supported systems might be attributed to a confinement effect. In line with this, they are moderately larger within CNF, which features wider channels. The EDX maps of AC-2, CNF-2, and CNT-2 also indicate that all Cu particles visualised are in contact with the Fe phase. In contrast, the contact among the metal is limited in $CNF_p$-2, with many Cu particles remaining isolated.

It was observed that the HA selectivity and $CO_2$ formation were inversely and directly proportional to the size of Cu particles. Since the Cu particle size was similar in the used CNT-2 and CNF-2, but the former generated less HA, less methanol, more hydrocarbons and, among those, fewer olefins, other structural characteristics shall also play a role, as expected in a bifunctional system. The more prominent formation of hydrocarbons, especially methane and aliphatic compounds, over CNT-2 seems related to the smaller size of the Fe particles in this sample. Since Cu is less dispersed than Fe, even if all Cu particles are closely located to Fe aggregates (as seen in the EDX maps for both samples), a significant portion of Fe remains isolated. Thus, it is advantageous that the FTS metal is more agglomerated to hinder hydrocarbon production, as in CNF-2. The high $CO_2$ selectivity observed over CP-2 was likely linked to the

EP 3 593 900 A1

large Cu particle size.

**Example 5: Effect of the CuFe ratio and synthesis method on CuFe/CNF catalysts**

The effect of the metal particle size and proximity were further studied by evaluating solids supported on this material and possessing different molar Cu/Fe ratios (1, 3 and 5) or still featuring a Cu/Fe ratio of 2 but being prepared by different synthesis methods.

In particular, when using the sol-gel route, a calcination step was included after impregnation and prior to reduction (CNF-2-C), or the two metals were incorporated in independent steps with intermediate reduction (Fe/Cu/CNF-2). Alternatively, deposition-precipitation using $Na_2CO_3$ as the precipitating agent (DP-CNF-2) or a wet deposition-reduction protocol using $NaBH_4$ as the reducing species (CR-CNF-2) were employed. According to ICP-OES, CNF-3 and CNF-5 had a slightly lower Cu loading than expected, which could be attributed to the hygroscopic nature of copper nitrate, used in larger amounts in these samples.

The measured molar Cu/Fe ratio and metals loading of the catalysts attained by distinct recipes were close to the nominal values (see **Table 3**), except for CR-CNF-2 and Fe/Cu/CNF-2. In the first case, the incomplete reduction of the metals by $NaBH_4$ in the precursor solution (particularly Fe) is put forward as the reason, while a mismatch in the Cu loading, due to incomplete precipitation, propagating to that of Fe seems to be the cause of the deviation for the second material. The surface area of all systems was similar to or slightly larger than that of the carrier (28-32 $m^2 g^{-1}$).

**Table** 3. Characterisation data for CNF-supported CuFe catalysts prepared with different molar Cu/Fe ratios and by different synthesis methods in reduced form.

| Catalysts | Bulk CuFe content[a] [wt.%] | Bulk molar Cu/Fe ratio[a] | Surface molar Cu/Fe ratio[b] | $S_{BET}$[c,d] [$m^2g^{-1}$] | $V_{pore}$[c,e] [$cm^3g^{-1}$] | $d_{Cu}$[c,f] [nm] |
|---|---|---|---|---|---|---|
| CNF-1 | 5.2 | 0.95/1 | 0.8 | 29 (22) | 0.10 (0.08) | 10.6 (10.6) |
| CNF-3 | 4.6 | 2.80/1 | - | 28 (23) | 0.10 (0.07) | 18.4 (10.4) |
| CNF-5 | 4.7 | 4.97/1 | 1.2 | 30 (23) | 0.10 (0.09) | 25.2 (19.2) |
| CNF-2-C | 5.1 | 2.04/1 | - | 32 (20) | 0.12 (0.09) | - (9.5) |
| Fe/Cu/CNF-2 | 5.1 | 2.22/1 | - | 28 (18) | 0.10 (0.08) | 11.6 (11.4) |
| CR-CNF-2 | 4.6 | 2.21/1 | 0.3 | 31 (24) | 0.09 (0.09) | 9.7 (22.8) |
| DP-CNF-2 | 4.8 | 2.10/1 | - | 29 (20) | 0.10 (0.08) | 10.9 (11.1) |

[a] ICP-OES.
[b] XPS.
[c] Data for the used samples in brackets.
[d] BET method.
[e] t-plot method.
[f] Determined from the (111) reflection in the XRD pattern using the Scherrer equation.

As intended, the product distributions attained for these catalysts at the same conversion level (**Figure 4b** and **Table 4**) are quite diverse.

Table 4: Performance data for the catalysts investigated.

| Catalyst[a] | WHSV ($cm^3 g_{cat}^{-1} h^{-1}$) | $STY_{HA[ROH]}$[a] ($g_{HA} g_{cat}^{-1} h^{-1}$) | $X_{CO}$ (%) | $S_{MeOH}$ (%) | $S_{HA}$ (%) | $S_{HC=}$ (%) | $S_{HC-}$ (%) | $S_{CO2}$ (%) |
|---|---|---|---|---|---|---|---|---|
| CNF-1 | 4,000 | 0.06 [0.10] | 49 | 4 | 9 | 8 | 54 | 25 |

14

(continued)

| Catalyst[a] | WHSV | $STY_{HA[ROH]}$[a] | $X_{CO}$ | $S_{MeOH}$ | $S_{HA}$ | $S_{HC=}$ | $S_{HC-}$ | $S_{CO2}$ |
|---|---|---|---|---|---|---|---|---|
| | (cm$^3$ g$_{cat}^{-1}$ h$^{-1}$) | (g$_{HA}$ g$_{cat}^{-1}$ h$^{-1}$) | (%) | (%) | (%) | (%) | (%) | (%) |
| | 24,000 | 0.15 [0.27] | 8 | 8 | 21 | 17 | 31 | 23 |
| CNF-3 | 4,000 | 0.07 [0.13] | 34 | 9 | 15 | 7 | 57 | 13 |
| | 24,000 | 0.14 [0.32] | 8 | 19 | 20 | 11 | 35 | 15 |
| CNF-5 | 4,000 | 0.08 [0.16] | 46 | 9 | 13 | 4 | 64 | 10 |
| | 32,000 | 0.12 [0.32] | 8 | 18 | 15 | 9 | 41 | 16 |
| CNF-2-C | 4,000 | 0.05 [0.09] | 38 | 5 | 10 | 10 | 59 | 16 |
| | 16,000 | 0.07 [0.13] | 8 | 9 | 14 | 11 | 42 | 24 |
| Fe/Cu/CNF-2 | 4,000 | 0.08 [0.12] | 29 | 5 | 12 | 12 | 50 | 21 |
| | 17,000 | 0.05 [0.07] | 8 | 7 | 16 | 18 | 27 | 32 |
| CR-CNF-2 | 4,000 | 0.02 [0.06] | 22 | 9 | 8 | 3 | 46 | 34 |
| | 10,000 | 0.02 [0.07] | 8 | 13 | 9 | 5 | 37 | 35 |
| DP-CNF-2 | 4,000 | 0.04 [0.07] | 23 | 8 | 12 | 5 | 63 | 12 |
| | 16,000 | 0.05 [0.16] | 8 | 17 | 12 | 4 | 32 | 35 |

[a] Reaction conditions: 543 K, 5 MPa, and molar $H_2$/CO = 2. HA = higher alcohols, MeOH = methanol, HC= = olefins, and HC- = paraffins.

The HA selectivity was similar for CNF-1 and CNF-3 and inferior compared to CNF-2 (21 *vs.* 31%). In both cases, the fraction of hydrocarbons produced remained overall constant (46-48%), although fewer olefins were formed on the Cu-rich catalyst. The decrease in HA was accompanied by a drop-in methanol production over CNF-1, which formed much more $CO_2$ overall. Over CNF-3, more $CO_2$ and methanol were generated at the expense of HA. CNF-5 formed a similar amount of methanol and $CO_2$ to CNF-3, but HA production was inferior due to an enhanced selectivity to hydrocarbons. As in the case of CNF-3, more paraffins were produced. This was mainly due to a boosted $CH_4$ formation.

The materials obtained by different synthesis methods all displayed a much less favourable product distribution than CNF-2. CNF-2-C and Fe/Cu/CNF-2 exhibited a higher HA selectivity than CR-CNF-2 and DP-CNF-2 (14-16 *vs.* 9-12%). Fe/Cu/CNF-2 had a comparable performance to CNF-1 and formed less methanol and paraffins, but more $CO_2$ than CNF-2-C. The catalysts synthesised by chemical reduction and deposition-precipitation mostly suffered from a high $CO_2$ and paraffins production and a reduced olefins generation. Both formed more methanol than HA. For all CNF-supported systems with Cu/Fe = 2, the trends in HA and olefins selectivities seemed coupled.

Correlating the catalytic performance to the composition, it is demonstrated that the methanol selectivity increased along with the Cu/Fe ratio up to a value of 3. CNF-5 exhibited a comparable selectivity to this alcohol to CNF-3, likely due to the much poorer Cu dispersion. Contrarily to the expectations, the hydrocarbon selectivity did not drop at higher Cu/Fe ratios but remained nearly constant. This is rationalised by a more enhanced dispersion of Fe when present in a lower amount and higher aggregation of the more abundant Cu, which leads to a lower contact among the metals. Accordingly, a larger portion of Fe acts alone favouring methanation and FTS. Since Cu and Fe in CNF-2-C feature similar dimensions to the metal phases in CNF-2, the separation between the two active metals, fostered by the intermediate calcination and formation of defined oxide compounds, is suggested to be the origin of the reduced HA formation and increased production of hydrocarbons and $CO_2$. The lack of intimacy among the two metals, induced by their sequential rather than simultaneous deposition, most likely rationalises the similar behaviour of Fe/Cu/CNF-2, since the dimensions of the metal aggregates are in the same range as in CNF-2-C and CNF-2. The requirement for metals proximity for a high HA synthesis efficiency is reinforced by the poor performance of CR-CNF-2, which features the largest Cu particle and a very limited contact with Fe.

This parameter is highlighted as the most important for catalyst design. Indeed, multiple materials are characterised by similarly small Fe and Cu size, but their HA selectivity spans through a wide range of values (**Table 4**). This finding justifies why very different optimal Cu/Fe ratios have been reported in relation to the best systems. The preferred value mostly fell within 1.5-3 but could also go up to 10 for bulk catalysts and was as low as 0.67 for the top performer among supported materials.

**Example 6: Effect of K promotion on CNF-supported Cu-Fe catalysts**

The impact of potassium as a promoter was studied on CNF-2. Three materials were prepared including different amounts of the alkali metal upon deposition of Cu and Fe so to reach nominal K/CuFe ratios of 0.005, 0.01 and 0.1. In addition, since the K loading is very small and falls below the detection limit of many characterisation techniques, it was attempted to gather insights into its role by varying the location and contact of this promoter with the active metals tailoring the synthesis method.

Hence, K was incorporated prior to or after the simultaneous deposition of Cu and Fe (CuFe/K/CNF-2-0.005 and K/CuFe/CNF-2-0.005) and along with Cu prior to Fe addition (Fe/CuK/CNF-2-0.005). The measured Cu/Fe ratio and Cu-Fe loading of the reduced catalysts were close to the nominal values (see **Table 5**), but the K loadings were systematically lower than expected, likely due to the presence of moisture in $K_2CO_3$.

**Table** 5. Characterisation data for CNF-supported KCuFe catalysts prepared with different K loadings and by different synthesis methods in reduced form.

| Catalysts | Cu-Fe content[a] [wt.%] | Bulk molar K/Cu-Fe ratio | Bulk molar Cu/Fe ratio[a] | Surface molar Cu/Fe ratio[b,c] | $S_{BET}$[c,d] [m²g⁻¹] | $V_{pore}$[c,e] [cm³g⁻¹] | $d_{Cu}$[c,i] [nm] |
|---|---|---|---|---|---|---|---|
| CNF-2-0.1 | Cu5.0 | 0.07 | 1.98/1 | - | 24 (0.4) | 0.09 (-) | 20.9 (9.8) |
| CNF-2-0.01 | 4.8 | 0.007 | 2.08/1 | - | 24 (4) | 0.09 (-) | 11.0 (8.8) |
| CNF-2-0.005 | 4.9 | 0.003 | 2.07/1 | 1.2 (0.3) | 24 (7) | 0.09 (0.02) | 11.4 (9.9) |
| K/CuFe/CNF-2-0.005 | 4.9 | 0.004 | 1.91/1 | 0.6 | 31 (28) | 0.11 (0.10) | 15.8 (14.7) |
| CuFe/K/CNF-2-0.005 | 5.0 | 0.003 | 1.91/1 | - | 29 (12) | 0.10 (0.03) | 19.3 (11.4) |
| Fe/CuK/CNF-2-0.005 | 4.4 | 0.004 | 2.02/1 | - | 28 (22) | 0.12 (0.10) | - |
| [a] ICP-OES.<br>[b] XPS.<br>[c] Data for the used samples in brackets.<br>[d] BET method.<br>[e] t-plot method.<br>[f] Size of Cu determined by Scherrer equation based on the Cu (111) crystalline phase. | | | | | | | |

When K was introduced prior to or after the active metals, the particles of both Cu and Fe had larger dimensions than in the case of CNF-2-0.005 and CNF-2-0.01, but Cu remained less aggregated and Fe formed larger particles with respect to CNF-2-0.1 (see Table 5). By simultaneously incorporating K and Cu before Fe, both active metals were highly dispersed.

The product distributions attained for these catalysts at the same conversion level are given in **Figure 4c** and **Table 6.**

Table 6: Performance data for the catalysts investigated.

| Catalyst | WHSV (cm³ g$_{cat}$⁻¹ h⁻¹) | $STY_{HA[ROH]}$[a] (g$_{HA}$ g$_{cat}$⁻¹ h⁻¹) | $X_{CO}$ (%) | $S_{MeOH}$ (%) | $S_{HA}$ (%) | $S_{HC=}$ (%) | $S_{HC-}$ (%) | $S_{CO2}$ (%) |
|---|---|---|---|---|---|---|---|---|
| CNF-2-0.1 | 4,000 | 0.02 [0.02] | 12 | 0.2 | 13 | 15 | 12 | 60 |
| | 8,000 | 0.03 [0.04] | 8 | 1 | 15 | 12 | 10 | 61 |
| CNF-2-0.01 | 4,000 | 0.09 [0.11] | 44 | 3 | 15 | 12 | 17 | 53 |
| | 16,000 | 0.22 [0.25] | 8 | 5 | 47 | 15 | 14 | 18 |

(continued)

| Catalyst | WHSV | STY$_{HA[ROH]}$[a] | $X_{CO}$ | $S_{MeOH}$ | $S_{HA}$ | $S_{HC=}$ | $S_{HC-}$ | $S_{CO2}$ |
|---|---|---|---|---|---|---|---|---|
| | (cm$^3$ g$_{cat}$$^{-1}$ h$^{-1}$) | (g$_{HA}$ g$_{cat}$$^{-1}$ h$^{-1}$) | (%) | (%) | (%) | (%) | (%) | (%) |
| CNF-2-0.005 | 4,000 | 0.18 [0.26] | 70 | 6 | 19 | 7 | 48 | 20 |
| | 32,000 | 0.28 [0.42] | 8 | 13 | 37 | 20 | 19 | 11 |
| CuFe/K/CNF-2-0.005 | 4,000 | 0.15 [0.21] | 61 | 5 | 16 | 12 | 31 | 36 |
| | 20,000 | 0.21 [0.28] | 8 | 9 | 30 | 20 | 23 | 18 |
| Fe/CuK/CNF-2-0.005 | 4,000 | 0.01 [0.02] | 8 | 5 | 11 | 16 | 46 | 21 |
| K/CuFe/CNF-2-0.005 | 4,000 | 0.01 [0.02] | 8 | 5 | 9 | 16 | 33 | 37 |
| KCuFe/SiO$_2$-BM[b] | 6,000 | 0.23 [0.32] | 53 | 23 | 38 | 11 | | 28 |
| CuFeMg[c] | 2,000 | 0.19 [0.28] | 57 | 16 | 33 | 39 | | 12 |
| Cu$_2$Fe-3DOM[d] | 2,000 | 0.15 [0.19] | 58 | 4 | 26 | 58 | | 12 |

[a] Reaction conditions: 543 K, 5 MPa, and molar $H_2$/CO = 2. HA = higher alcohols, MeOH = methanol, HC= = olefins, and HC- = paraffins. [b] Reference catalyst, data retrieved from Fuel Process. Technol. 2017, 159, 436-441. [c] Reference catalyst, data retrieved from Catal. Sci. Technol. 2013, 3, 1324-1332. [d] Reference catalyst, data retrieved from ACS Catal. 2017, 7, 5500-5512.

Reduced CNF-2-0.005 and K/CuFe/CNF-2-0.005 were additionally analysed by STEM-EDX. In the former (see **Figure 4c**), the particles were mostly visualised inside the CNF channels. The average Cu and Fe particle sizes were calculated at 15.0 and 5.9 nm, respectively.

Compared to the K-free CNF-2, the interaction between Cu and Fe was found to be superior, not only because of the better contact, as represented by the two particles detected at the pore mouth of a nanofiber, but also because less isolated Fe particles were observed. Based on fringe analysis of the corresponding HRTEM image, the Cu(111) and Fe(011) planes were identified.

When K was incorporated subsequently to Cu and Fe (**Figure 4b**) large particles of Cu (17.8 nm) and Fe (8.8 nm) formed, which featured a poorer interaction, both in terms of contact area and number of neighbouring aggregates.

To shed light onto the electronic effects of K, CNF-2-0.005 and K/CuFe/CNF-2-0.005 were characterised by $H_2$-TPD along with selected K-free samples, $i.e.,$ the counterpart of the former, CNF-2, and the poorly performing CR-CNF-2. $H_2$-TPD has been applied by several groups to investigate the $H_2$ adsorption ability of the metals and has always been performed under ambient conditions. In this example, the measurements have been conducted under the same pressure as it was applied in the tests. Specifically, the reduced catalysts were reactivated in a diluted $H_2$ flow at 573 K and 0.5 MPa for 3 h, as done in the catalytic reactor prior to the runs, exposed to 60 vol% of $H_2$ at 5 MPa and 543 K as upon reaction, flushed with He at the same pressure and temperature, and then heated in the inert stream up to 1103 K.

Testing of the promoted catalysts indicated that the simultaneous addition of small amounts of K to Cu and Fe during synthesis (CNF-2-0.005 and CNF-2-0.01) increased the selectivity towards HA, alkenes and $CO_2$, and reduced that towards methanol and alkanes. The HA selectivity reached 37% over CNF-2-0.005 and matched the selectivity of the state of the art CuFe catalyst (47%) over CNF-2-0.01. For the latter, the $CO_2$ selectivity raised to 18%, which is still much lower than for the reference material (28%). It was noteworthy that the selectivity to alkenes surpassed that of alkanes (15 $vs.$ 14%). The addition of greater K amounts (CNF-2-0.1) led to the predominant production of $CO_2$ (60%) and the HA selectivity was lowered to 15%.

The alkali effect of minimising methanol formation was more pronounced at higher contents, though. The methanol selectivity decreased from 12 to 0.3%. Since stronger promotion also reduced the CO conversion, the highest $STY$ of HA was obtained over CNF-2-0.005 (0.28 g$_{HA}$ g$_{cat}$$^{-1}$ h$^{-1}$). When K was introduced prior to the deposition of the active metals, the HA selectivity was similar to that of the K-free CNF-2 (30 %) but more $CO_2$ was formed (18 $vs.$ 6%) at the expense of paraffins and methanol. When the promoter was added after Cu and Fe, the HA selectivity reduced drastically to 9% and $CO_2$ was the most abundant product (37%). Fe/CuK/CNF-2-0.005 also exhibited a low selectivity towards HA but generated more alkanes (46%) and less $CO_2$(21%).

STEM-EDX and HRTEM were conducted to assess the metals morphology, distribution and speciation in CNF-2-0.005 after the reaction. The mapping pointed to a higher fraction of particles located at the fibres' outer surface compared to the reduced solid. The particle size and interaction between Cu and Fe were preserved. A core-shell structure was detected in Fe-containing particles. Based on HRTEM, these comprised amorphous carbon in the outer

layer and $Fe_2C$ as the inner core. The (111) plane of this carbide was identified upon fringe analysis. Based on XPS, the surface Cu/Fe ratio was lowered from 1.6 to 1.3. This change is attributed to the moderately increased agglomeration of Fe (5.9 to 6.7 nm).

Overall, the catalytic and characterisation data for promoted systems corroborate the conclusions drawn for the K-free materials with respect to the fact that higher HA selectivity is obtained if the Cu particles are around 10 nm in size, Fe is not too dispersed, and a good interaction is established between the two active metals. The addition of K in small quantities enables the formation of a slightly more aggregated Fe phase, which has similar or even improved contact with Cu, minimising alkanes formation and boosting HA and olefins production. Thus, K plays an important structural role. In addition, electronic effects are also likely. $H_2$-TPD suggests an enhanced but more labile binding of $H_2$ to the metals, which shall help slowing the kinetics of hydrogenation reactions. This further substantiates the scarce relevance of the surface Cu/Fe ratio as a stand-alone descriptor.

**Example 7: Kinetic and stability analysis over the best K-promoted CuFe/CNF catalyst**

Kinetic aspects of the synthesis of HA were explored over the catalyst providing the highest STY of HA, CNF-2-0.005. Measurements were conducted in a wide range of temperatures, pressures, molar $H_2$/CO ratio and *WHSV.*

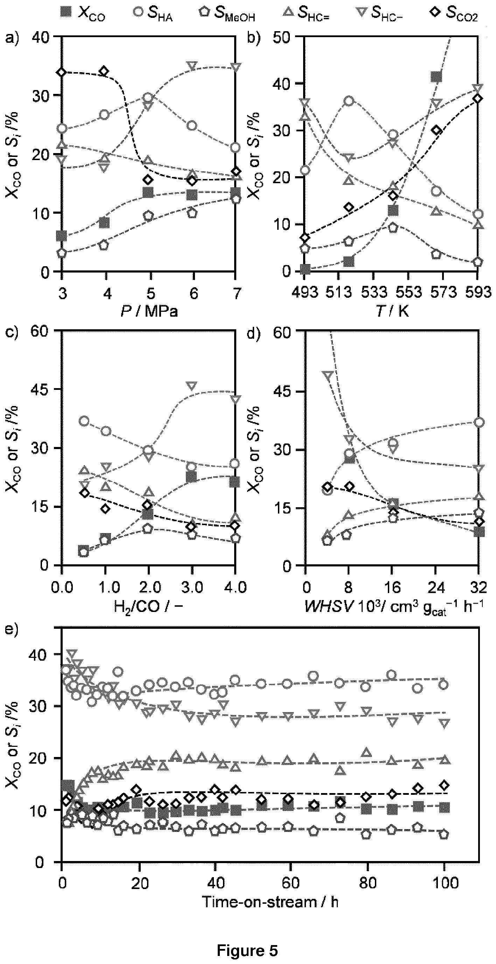

An increase in total pressure from 3 to 7 MPa (**Figure 5a**) doubled the CO conversion (6 *vs.* 12%), while the HA selectivity increased up to 5 MPa and decreased at higher pressures. Below 5 MPa, more $CO_2$ and olefins were produced, while above that pressure the formation of methanol and alkanes (mainly $CH_4$) was favoured. The synthesis of HA is an equilibrium reaction in which the number of moles of the products is inferior to that of the reactants and is thus expected to be favoured at high pressures. This also holds for methanol and hydrocarbons synthesis.

In order to study the temperature dependence (**Figure 5b**), its value was step-wise increased by 25 K in the range of 493-593 K. The CO conversion raised exponentially, while optimal selectivity to HA (37%) and methanol (10%) was reached at 518 K and 543 K, respectively.

The formation of olefins was inhibited at high temperature, while that of $CO_2$ was favoured due to a higher water-gas shift (WGS) activity, as expected.

The molar $H_2$/CO ratio was varied between 0.5 and 4 (**Figure 5c**). A higher relative $H_2$ concentration enhanced the CO conversion and the hydrocarbons selectivity while suppressing the selectivity towards HA and $CO_2$. The formation of alkanes, methane, in particular, was enhanced, while alkenes were produced to a lower extent. The trends observed for hydrocarbons and methanol are in line with the greater availability of $H_2$.

Concerning the *WHSV*, a higher value reduced the CO conversion, as expected in view of the shorter residence time, but was beneficial to the selectivity towards HA, methanol, and olefins, the amount of which increased at the expenses of paraffin and $CO_2$ (**Figure 5d**). It is worth noting that the boost in HA selectivity was higher than for the other two compounds. These findings indicate that hydrogenation of carbon species and alcohol dehydration have slower kinetics, while CO insertion occurs quickly, provided that the two catalytic function where the alkyl and CO species are formed are spatially close. Based on these observations, the differences in product distributions derived for the variously supported catalysts could have been affected by the *WHSV* applied in the individual tests, which was varied in a quite large range to attain comparable activity levels. Still, the contribution by the operating conditions would mostly affect materials evaluated at less than 8000 $h^{-1}$ (*e.g.,* the anyway very inefficient AC-2 catalyst), since the HA selectivity is mostly altered in this interval. For CNT- and CNF-supported systems, diffusion properties related to the presence of particles of metals of a distinct size, differently occluding the channels, and of coke deposits, as well as the fraction of particles in or outside the tubes, will contribute to the selectivity fingerprint of the materials to some extent as well. Ascertaining this aspect in a quantitative manner is very difficult and lies out of the scope of this work.

Since the stability of HA synthesis catalysts has infrequently been evaluated, CNF-2-0.005 was assessed in a 100-h catalytic run (**Figure 5e**). Exploiting the knowledge gained from the kinetic study, the $H_2$/CO ratio was reduced from 2 to 1.5 and the *WHSV* was increased from 16000 to 32000 $h^{-1}$ to boost the HA selectivity, keeping the pressure at the optimal value of 5 MPa. To compensate for the concomitant drop in CO conversion under these conditions, the temperature was ever so slightly increased (from 543 to 548 K). This allowed to maintain the HA selectivity very close to the maximum (37 *vs.* 39%) at a CO conversion of 14%. In the first 10 h of the test, the selectivity to alkanes and alkenes appreciably decreased and increased, respectively, and the CO conversion declined to 11%. Thereafter, the catalytic properties remained practically undisturbed until the end of the test. The initial equilibration phase is likely associated with the formation of iron carbides.

An $STY_{HA}$ of 0.53 $g_{HA}$ $g_{cat}^{-1}$ $h^{-1}$ was calculated for the stable period and was double-that of the benchmark CuFe catalyst using bimodal silica as support and K as a promoter (0.23 $g_{HA}$ $g_{cat}^{-1}$ $h^{-1}$, **Table 6**).

**Claims**

1. Catalyst composition comprising copper and iron on a support for use in a process for the synthesis of higher alcohols from a syngas feed stream comprising hydrogen and carbon monoxide, the catalyst composition being **characterised in that** the support is a carbon-containing support selected from carbon nanofibers and/or carbon nanotubes, **in that** the total content of iron and copper is ranging from 1 to 10 wt% based on the total weight of the catalyst composition and as determined by inductively coupled plasma optical emission spectroscopy, **in that** the Cu/Fe ratio is ranging from 0.5:1 to 5:1, and **in that** the carbon-containing support has a hollow-core structure with an inner diameter of at least 17 nm as determined according to $N_2$ sorption analysis.

2. Catalyst composition according to claim 1, **characterised in that** the carbon-containing support is carbon nanofibers; and/or **in that** the carbon-containing support has hollow-core structure with an inner diameter of at least 20 nm as determined according to $N_2$ sorption analysis, preferably of at least 25 nm, more preferably of at least 30 nm.

3. Catalyst composition according to any one of claims 1 or 2, **characterised in that** the carbon-containing support is carbon nanofibers selected from platelet-type carbon nanofibers and conical platelet-type carbon nanofibers; with preference, the carbon-containing support is conical platelet-type carbon nanofibers.

4. Catalyst composition according to any one of claims 1 to 3, **characterised in that** the Cu particle size is at least 7 nm as determined from the (111) reflection in an XRD pattern using the Scherrer equation, preferably at least 8 nm, more preferably at least 9 nm; and/or the Cu particle size is at most 35 nm as determined from the (111) reflection in a XRD pattern using the Scherrer equation, preferably at most 20 nm, more preferably at most 11 nm.

5. Catalyst composition according to any one of claims 1 to 4, **characterised in** the total metal loading is ranging from 2.0 to 8.0 wt% based on the total weight of the catalyst composition and as determined by inductively coupled plasma optical emission spectroscopy, preferably from 4.5 to 5.5 wt%.

6. Catalyst composition according to any one of claims 1 to 5, **characterised in that** the Cu/Fe bulk molar ratio is ranging from 1:1 to 4:1, preferably from 1.2:1 to 3:1.

7. Catalyst composition according to any one of claims 1 to 6, **characterised in that** the composition further comprises at least one promoter; preferably at least one promoter is selected from alkali and alkaline earth metal, more preferably at least one promoter is selected from alkali metal, even more preferably at least one promoter is potassium.

8. Catalyst according to claim 7, **characterised in that** the content of at least one promoter is ranging from 0.001 to 0.5 wt% based on the total weight of the catalyst composition and as determined by inductively coupled plasma optical emission spectroscopy, preferably ranging from 0.001 to 0.1 wt%.

9. Method to produce a catalyst composition according to any one of claim 1 to 8 **characterised in that** the method comprises the following steps:

   i. Co-precipitating iron and copper together, optionally with at least one promoter, and re-dissolving them with a complexing agent to form a mixture;
   ii. Depositing the mixture on the carbon-containing support to obtain a slurry;
   iii. Drying the slurry and activating the dried material through reduction to obtain a reduced catalyst composition;
   iv. Optionally calcining the dried catalyst composition to obtain a calcined catalyst composition.

10. The method of claim 9 **characterised in that** the activation step iii) is performed in diluted $H_2$ at a temperature above 600 K

11. Process for the synthesis of higher alcohols from a syngas feed stream comprising hydrogen and carbon monoxide, **characterised in that** it comprises the following steps:

   a) providing a syngas feed stream comprising hydrogen and carbon monoxide;
   b) providing a catalyst composition according to any one of claims 1 to 8;
   c) putting the syngas feed stream into contact with the catalyst composition at a reaction pressure ranging from 1 to 10 MPa and a reaction temperature ranging from 443 K (169.85°C) to 653 K (379.85°C); and
   d) recovering the effluent containing higher alcohols.

12. The process of claim 11 **characterised in that** step c) is conducted at a *WHSV* of at least 1,000 cm$^3$ g$_{cat}^{-1}$ h$^{-1}$, preferably of at least 2,000 cm$^3$ g$_{cat}^{-1}$ h$^{-1}$.

13. The process of claim 11 or 12, **characterised in that** the syngas feed stream is selected to have a molar H$_2$/carbon oxide ranging from 0.5:1 to 12.0:1, preferably ranging from 1.0:1 to 4:1.

14. The process of any one of claim 11 to 13, **characterised in that**:

   - the pressure is ranging from 3 to 7 MPa , preferably from 4 to 6 MPa; and/or
   - the temperature is ranging from 493 K (219.85°C) to 553 (279.85°C), preferably from 513 K (239.85°C) to 548 K (274.85°C).

15. Use of a Cu-Fe-based catalyst composition in a process for the synthesis of higher alcohols from syngas, the use being **characterised in that** the catalyst composition is according to any one of claim 1 to 8.

Figure 1

Figure 2

Figure 3

Figure 4

Figure 5

Europäisches
Patentamt

European
Patent Office

Office européen
des brevets

**EUROPEAN SEARCH REPORT**

Application Number

EP 18 30 5922

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GENOVESE CHIARA ET AL: "Electrocatalytic conversion of CO2 on carbon nanotube-based electrodes for producing solar fuels", JOURNAL OF CATALYSIS, vol. 308, 4 October 2013 (2013-10-04), pages 237-249, XP028782874, ISSN: 0021-9517, DOI: 10.1016/J.JCAT.2013.08.026 | 1-8 | INV. B01J21/18 B01J23/72 B01J23/745 B01J23/78 B01J37/02 B01J37/03 B01J37/16 |
| A | * the whole document * <br> * Page 240-241: "2.1 Preparation of the electrocatalyst " * <br> * Pages 243-244: "3.3 Use of bimetallic metal nanoparticles " * <br> * table 2 * <br> * figure 6b * | 9,10 | B01J35/06 C07C29/154 C07C29/156 C07C31/02 |
| X | SHI XINPING ET AL: "Synergistic effect of nitrogen-doped carbon-nanotube-supported Cu-Fe catalyst for the synthesis of higher alcohols from syngas", FUEL, vol. 210, 31 August 2017 (2017-08-31), pages 241-248, XP085270245, ISSN: 0016-2361, DOI: 10.1016/J.FUEL.2017.08.064 | 1-8, 11-15 | |
| Y | * the whole document * <br> * Page 242: "2.1 Materials and catalyst preparation" and "2.3 Catalyst testing" * | 1-8, 11-15 | TECHNICAL FIELDS SEARCHED (IPC) <br> B01J <br> C07C |
| A | * Pages 242-244: " 3.1 Structural and textural properties" * <br> * Pages 245-246: "3.5.1 Effects of support, Cu/Fe ratio and metal loading" * | 9,10 | |

-/--

The present search report has been drawn up for all claims

| Place of search | Date of completion of the search | Examiner |
|---|---|---|
| Munich | 4 January 2019 | Gosselin, Daniel |

EPO FORM 1503 03.82 (P04C01)

Europäisches
Patentamt

European
Patent Office

Office européen
des brevets

## EUROPEAN SEARCH REPORT

Application Number

EP 18 30 5922

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Xinping Shi ET AL: "Synergistic effect of nitrogen-doped carbon-nanotube-supported Cu-Fe catalyst for the synthesis of higher alcohols from syngas", Fuel, 15 December 2017 (2017-12-15), pages 241-248, XP055538228, DOI: 10.1016/j.fuel.2017.08.064 Retrieved from the Internet: URL:https://ars.els-cdn.com/content/image/1-s2.0-S0016236117310499-mmc1.docx | 1-8, 11-15 | |
| Y | * the whole document * * tables S2-S3 * | 1-8, 11-15 | |
| A | | 9,10 | |
| Y | US 2012/283342 A1 (DALAI AJAY KUMAR [CA] ET AL) 8 November 2012 (2012-11-08) | 1-8, 11-15 | |
| A | * the whole document * * paragraphs [0014], [0219], [0220], [0234], [0235] * | 9,10 | |
| A | NELLY M. RODRIGUEZ ET AL: "Carbon Nanofibers: A Unique Catalyst Support Medium", JOURNAL OF PHYSICAL CHEMISTRY, vol. 98, no. 50, 1 December 1994 (1994-12-01), pages 13108-13111, XP055538059, US ISSN: 0022-3654, DOI: 10.1021/j100101a003 * the whole document * | 1-15 | TECHNICAL FIELDS SEARCHED (IPC) |

-/--

The present search report has been drawn up for all claims

| Place of search | Date of completion of the search | Examiner |
|---|---|---|
| Munich | 4 January 2019 | Gosselin, Daniel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 18 30 5922

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ERIC VAN STEEN ET AL: "Comparison of preparation methods for carbon nanotubes supported iron Fischer-Tropsch catalysts", CATALYSIS TODAY, vol. 71, no. 3-4, 1 January 2002 (2002-01-01), pages 327-334, XP055538057, AMSTERDAM, NL ISSN: 0920-5861, DOI: 10.1016/S0920-5861(01)00459-X * the whole document * * Page 328: "2. Experimental " * * Pages 329-330: "Results and discussion" * * Page 333: "4. Conclusions" * * tables 1-2 * | 1-10 | |

TECHNICAL FIELDS
SEARCHED (IPC)

The present search report has been drawn up for all claims

| Place of search | Date of completion of the search | Examiner |
|---|---|---|
| Munich | 4 January 2019 | Gosselin, Daniel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 3

**EP 3 593 900 A1**

## ANNEX TO THE EUROPEAN SEARCH REPORT
## ON EUROPEAN PATENT APPLICATION NO.

EP 18 30 5922

04-01-2019

| Patent document cited in search report | Publication date | Patent family member(s) | Publication date |
|---|---|---|---|
| US 2012283342 A1 | 08-11-2012 | NONE | |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2257366 A
- US 7279138 B

**Non-patent literature cited in the description**

- **RUN XU et al.** Influence of Promoters on Catalytic Properties of Cu-Mn-Fe/ZrO2 Catalysts for Alcohols Synthesis. *React. Kinet. Catal. Lett.,* 2004, vol. 81, 91-98
- **M. LIN et al.** CO Hydrogenation to Mixed Alcohols over Co-precipitated Cu-Fe Catalysts. *Catal. Commun.,* 2008, vol. 9, 1869-1873
- **MINGYUE DING et al.** Influence of Manganese Promoter on Co-Precipitated Fe-Cu Based Catalysts for Higher Alcohols Synthesis. *Fuel,* 2013, vol. 109, 21-27
- **KANG XIAO et al.** Structural Evolution of CuFe Bimetallic Nanoparticles for Higher Alcohol Synthesis. *J. Mol. Catal. A: Chem.,* 2013, vol. 378, 319-325
- **ZHENGHONG BAO et al.** Higher Alcohol Synthesis over Cu-Fe Composite Oxides with High Selectivity to C2+OH. *Journal of Energy Chemistry,* 2013, vol. 22, 107-113
- **MINGYUE DING et al.** Copper-Iron Supported Bimodal Pore Catalyst and is Application for Higher Alcohol Synthesis. *Catalysis Today,* 2014, vol. 234, 278-284
- **XINYOU HAN et al.** Effects of Metal Promotion on CuMgFe Catalysts Derived from Layered Double Hydroxides for Higher Alcohol Synthesis via Syngas. *RSC Adv.,* 2015, vol. 5, 51868-51874
- **XINPING SHI et al.** Synergistic Effect of Nitrogen-doped Carbon-nanotube-supported Cu-Fe Catalyst for the Synthesis of Higher Alcohols from Syngas. *Fuel,* 2017, vol. 210, 241-248
- *Fuel Process. Technol.,* 2017, vol. 159, 436-441
- *Catal. Sci. Technol.,* 2013, vol. 3, 1324-1332
- *ACS Catal.,* 2017, vol. 7, 5500-5512